# EUROPÄISCHE PATENTSCHRIFT

(11) **EP 3 765 140 B1**
(45) Veröffentlichungstag und Bekanntmachung des Hinweises auf die Patenterteilung: **30.03.2022**
(21) Anmeldenummer: 19708437.9
(22) Anmeldetag: 26.02.2019
(51) Int. Cl.: A61N 1/05, A61N 1/36

(54) **IMPLANTAT IN ART EINER WICKELMANSCHETTEN-ELEKTRODENANORDNUNG**
IMPLANT COMPRISING A CUFF-TYPE ELECTRODE ARRANGEMENT
IMPLANT COMPRENANT UN ARRANGEMENT D'ÉLECTRODES DE TYPE À MANCHETTE

(30) Priorität: 16.03.2018 DE 102018204036
(43) Veröffentlichungstag der Anmeldung: 20.01.2021
(73) Patentinhaber: Neuroloop GmbH, 79110 Freiburg (DE)
(72) Erfinder: BORETIUS, Tim, 79098 Freiburg (DE); PLACHTA, Dennis, 79279 Vörstetten (DE); KIMMIG, Fabian, 79110 Freiburg (DE)
(74) Vertreter: Rösler, Uwe
(86) Internationale Anmeldenummer: PCT/EP2019/054643
(87) Internationale Veröffentlichungsnummer: WO 2019/174900

(56) Entgegenhaltungen:
- US-A1- 2004 015 204
- US-A1- 2006 030 919
- US-A1- 2015 374 296
- SHARIF KHAN ET AL: "Reliability of spring interconnects for high channel-count polyimide electrode arrays", JOURNAL OF MICROMECHANICS & MICROENGINEERING, INSTITUTE OF PHYSICS PUBLISHING, BRISTOL, GB, Bd. 28, Nr. 5, 8. März 2018 (2018-03-08), Seite 55007, XP020326536, ISSN: 0960-1317, DOI: 10.1088/1361-6439/AAAF2C [gefunden am 2018-03-08]

## Beschreibung

### Technisches Gebiet

Die Erfindung bezieht sich auf ein medizinisches Implantat in Art einer Wickelmanschetten-Elektrodenanordnung, kurz Cuff-Elektrode, geeignet zur extravasalen oder extraneuronalen Befestigung längs eines intrakorporalen Gefäßes oder eines Nervenfaserbündels, die ein flexibles, biokompatibles, folienartiges Trägersubstrat aufweist, das in einem ersten Bereich durch Wickeln um eine Wickelachse die Gestalt eines Tubus mit einer Tubuslänge annimmt, der einen geraden, zylinderförmigen Hohlraum umfasst, der von einer Oberfläche des Trägersubstrats radial zur Wickelachse begrenzt ist, an der wenigstens eine Elektrodenoberfläche angebracht ist, die über wenigstens eine innerhalb des Trägersubstrates integrierte elektrische Leitung mit einer nicht flexiblen Kontaktanordnung verbunden ist, an der die wenigstens eine elektrische Leitung mit einer elektrischen Zu- und/oder Ableitung verbunden ist, die zu einer zum Implantat separat ausgebildeten, implantierbaren elektrischen Versorgungseinheit führt.

### Stand der Technik

Cuff-Elektroden der vorstehend genannten Gattung werden zumeist zur Erfassung sowie auch Applizierung elektrischer Signale von bzw. an intrakorporale Gefäße, insbesondere an Nervenstränge angebracht. Cuff-Elektroden weisen hierzu ein flexibles, biokompatibles, folienartig ausgebildetes Trägersubstrat auf, an dessen dem Nervenstrang zugewandten Oberfläche wenigstens eine, vorzugsweise eine Vielzahl von Elektroden angebracht ist, die es gilt in innigen körperlichen Kontakt mit der Oberfläche eines Nervenstranges zu fügen.

Eine derartige Cuff-Elektrode ist in der Druckschrift WO 2016/055512 A1 offenbart. Das Trägersubstrat der Cuff-Elektrode besteht aus einer Polyimid-Folie, der zum Zwecke einer zumindest abschnittsweisen selbstständigen Aufwicklung um eine Wickelachse eine mechanische Folienvorspannung eingeprägt worden ist. Durch den Wickelvorgang der Cuff-Elektrode um den Nervenstrang gelangen die einzelnen Elektroden der Cuff-Elektrode in unmittelbaren Oberflächenkontakt mit dem Epineurium eines Nervenfaserbündels. Die Cuff-Elektrode fügt sich im Wege einer Kraft-beaufschlagten Formschlussverbindung an die zylinderförmige Außenfläche des Nervenfaserbündels an, wobei die durch den Aufroll- bzw. Wickelvorgang in gegenseitige Überlappung gelangenden Trägersubstratbereiche lose gleitend aufeinander liegen.

Die von den einzelnen Elektroden der Cuff-Elektrode abführenden elektrischen Leitungen verlaufen jeweils elektrisch isoliert innerhalb der Polyimid-Folie und enden an einem Seitenkantenbereich der Polyimid-Folie, der räumlich beabstandet zu dem gewickelten Bereich der Cuff-Elektrode liegt und an dem die Enden der elektrischen Leitungen jeweils über eine elektrische Kontaktanordnung mit elektrischen Zu- und Ableitungen verbunden sind, über die die Cuff-Elektrode mit einer implantierbaren elektrischen Versorgungseinheit, die separat zur Cuff-Elektrode intrakorporal verortet ist, verbunden ist.

Aufgrund der naturgegebenen Empfindlichkeit von Nervenfasern gegenüber äußeren mechanischen Einflüssen gilt es zum einen die durch die Cuff-Elektrode auf das Nervenfaserbündel einwirkenden mechanischen Belastungen so gering wie möglich zu halten, zum anderen gilt es darauf zu achten, dass die Cuff-Elektrode stabil genug das Nervenfaserbündel umfasst, um eine möglichst langlebige und ortsfeste Fügung der Cuff-Elektrode längs des Nervenfaserbündels zu gewährleisten.

Praktischen Erfahrungen beim Einsatz von Cuff-Elektroden zeigen zum einen, dass durch die lose Wicklung des zum Teil mehrlagig um ein Nervenfaserbündel anliegenden Trägersubstrats eine dynamische radiale Aufweitung der Cuff-Elektrode möglich ist, wodurch sich die Cuff-Elektrode naturgegebenen Formänderungen des Nervenfaserbündels anzupassen vermag, andererseits vermögen äußere, auf die Cuff-Elektrode einwirkende Kräfte ihre Wickelgeometrie signifikant zu deformieren, wodurch das Nervenfaserbündel erheblichen mechanischen Belastungen ausgesetzt sein kann, die zu irreversiblen Schädigungen führen können.

Die Druckschrift DE 44 33 111 A1 beschreibt eine Cuff-Elektrode, die aus einem flexiblen, mehrschichtigen Substrat aus nichtleitendem Silikon, auf dem sich erhabene Elektroden aus leitfähigem Silikon befinden. Die zu den Elektroden führenden Leiterbahnen verlaufen zwischen den nicht leitfähigen Schichten und bestehen ebenfalls aus leitfähigem Silikon. Durch die Einstellung verschiedener Vorspannungen im Mehrschichtaufbau der Cuff-Elektrode entsteht die aufgerollte Form der Cuff-Elektrode.

Die Druckschrift US 2015/0374296 A1 beschreibt ein Cuff-Elektrodenanordnung, die auch als Wickelelektrode ausgebildet sein kann und einen elektronischen Schaltkreis vorsieht, der über eine Vielzahl einzelner Elektronikkomponenten verfügt. Der elektrische Schaltkreis ist an der Außenwand der Cuff-Elektrode und/oder an einer entsprechenden Befestigungsstrukturen angebracht.

Aus dem Artikel von Sharif Khan et al, "Reliability of spring interconnects for high channel-count polyimide electrode arrays", Journal of Micromechanics & Microengineering, Inst. of Physics Publishing, Bristol, GB Bd. 28, Nr. 5, 8, März 2018 (2018-03-08), Seite 55007, XP020326536, ISSN:0960-1317, DOI: 10.1088/1361-6439/AAAF2C, geht ein neurales Implantat mit einer polyimiden Elektrodenanordnung und Federkontakten hervor, wobei die elektrische Kontaktierungstechnik zwischen den Elektrodenbereichen nicht auf ein und dem gleichen Substrat erfolgt..

### Darstellung der Erfindung

Der Erfindung liegt die Aufgabe zugrunde eine Cuff-Elektrode, die ein flexibles, biokompatibles, folienartiges Trägersubstrat aufweist, das in einem ersten Bereich durch Wickeln um eine Wickelachse die Gestalt eines Tubus annimmt, der einen geraden, zylinderförmigen Hohlraum umfasst, der von einer Oberfläche des Trägersubstrats radial zur Wickelachse begrenzt ist, an der wenigstens eine Elektrodenoberfläche angebracht ist, die über wenigstens eine innerhalb des Trägersubstrates integrierte elektrische Leitung mit einer nicht flexiblen Kontaktanordnung verbunden ist, an der die elektrische Leitung mit einer elektrischen Zu- und/oder Ableitung verbunden ist, die zur einer zum Implantat separat ausgebildeten, implantierbaren elektrischen Versorgungseinheit führt, derart weiterzubilden, so dass mit möglichst einfachen Mitteln für eine möglichst gleichmäßige und schonende Kraft- bzw. Druckeinwirkung durch die Cuff-Elektrode auf das Nervenfaserbündel gesorgt werden soll. Die soll insbesondere für jene Fälle gelten, in denen die Cuff-Elektrode durch längs der elektrischen Zu- und/oder Ableitung wirkenden Zugkräften belastet wird, wie sie bspw. durch körpereigene Bewegungen verursacht sein können.

Die Lösung der der Erfindung zugrundeliegenden Aufgabe ist im Anspruch 1 angegeben. Den Lösungsgedanken weiterbildende Merkmale sind Gegenstand der Unteransprüche sowie der weiteren Beschreibung zu entnehmen.

Lösungsgemäß zeichnet sich das in Art einer Wickelmanschetten-Elektrodenanordnung ausgebildete medizinische Implantat gemäß den Merkmalen des Oberbegriffes des Anspruches 1 dadurch aus, dass die Kontaktanordnung mit dem Trägersubstrat längs eines formstabilen Fügebereiches unmittelbar fest gefügt ist. Der Fügebereich besitzt eine räumliche Längserstreckung, die parallel zur Wickelachse orientiert ist, wobei die Kontaktanordnung in orthogonaler Projektion zur Wickelachse mit dem zu einem Tubus gewickelten ersten Bereich des Trägersubstrates überlappt und einen plättchenförmigen Träger aufweist, an dem die wenigstens eine elektrische Leitung mittels eines elektrischen Kontaktes kontaktiert ist, der elektrisch mit jeweils einer auf dem plättchenförmigen Träger aufgebrachten Elektrode verbunden ist, mit der die elektrische Zu- und/oder Ableitung (15) elektrisch verbunden ist. Ferner ist die elektrische Zu- und/oder Ableitung samt des plättchenförmigen Trägers von einem elastischen Material umschlossen, das im Fügebereich fluiddicht an das Trägersubstrat gefügt ist.

Der lösungsgemäßen Ausbildung der Cuff-Elektrode, die eine besondere Ausbildung und Anbringung der Kontaktanordnung relativ zum Trägersubstratbereich der Cuff-Elektrode betrifft, der durch einen Aufroll- bzw. Wickelvorgang die Form eines Tubus annimmt und im implantierten Zustand um ein Nervenfaserbündel lokal anliegt, liegen Erfahrungen zugrunde, die mit einer an sich bekannten Cuff-Elektrode gemäß der Druckschrift WO 2016/055512 A1 gewonnen wurden. Bei der bekannten Cuff-Elektrode ist jener Bereich des folienartig ausgebildeten Trägersubstrates, der sich unmittelbar einstückig an den zu einem Tubus gewickelten ersten Bereich des Trägersubstrates anschließt, im Wesentlichen band- bzw. streifenförmig ausgebildet. Längs des bandförmigen Trägersubstratbereiches verlaufen sämtliche mit den Elektroden der Cuff-Elektrode verbundenen elektrischen Leiter. Der bandförmige Trägersubstratbereich weist eine im Wesentlichen parallel zur Wickelachse orientierte Längserstreckung auf und ist über einen rechtwinklig geformten schmalen Bandabschnitt mittig lokal an dem zu einem Tubus gewickelten Trägersubstratbereich verbunden. Bei längs des parallel zur Wickelachse orientierten, bandförmig ausgebildeten Trägersubstrates wirkenden Zug- oder Schubkräften kommt es zu einer asymmetrischen Krafteinwirkung auf den zu einem Tubus gewickelten Trägersubstratbereich. Die hierdurch entstehenden Belastungszustände können längs zur Wickelachse auf der einen Seite des gewickelten Trägersubstratbereiches zu einer enger werdenden Zusammenwicklung und einer damit einhergehenden Einschnürung und auf der gegenüberliegenden Seite des gewickelten Trägersubstratbereiches zu einer Aufweitung und einer damit einhergehenden lokalen Abwicklung führen, wodurch der innenliegende Nervenstrang einer signifikant heterogenen Druck- bzw. Krafteinwirkung ausgesetzt ist.

Die lösungsgemäße Ausbildungsform hingegen vermeidet derartige asymmetrische, auf den Nervenstrang wirkende Belastungssituationen, indem die aus einem nicht flexiblen Material, bspw. aus einer Keramik, gefertigte Kontaktanordnung als eine Art Kräftekoppler dient, der längs der elektrischen Zu- und/oder Ableitung wirkende Zug- und/oder Schubkräfte möglichst gleichmäßig auf den Trägersubstratbereich, der zu einem Tubus gewickelt und manschettenartig um einen Nervenfaserstrang anliegt, verteilt weiterleitet. Eine asymmetrische Wickelgeometrie, bei der ein Ende des Tubus einen kleinen und das andere Ende einen vergrößerten Durchmesser erfährt, wird hierdurch vermieden. Vorzugsweise erfolgt die Krafteinleitung über die Kontaktanordnung auf den zu einem Tubus gewickelten Trägersubstratbereich gleichmäßig längs der gesamten axialen Längserstreckung des Tubus. Auf diese Weise können jegliche auf den Nervenstrang einwirkende Scherkräfte auf den Nervenstrang vermieden werden. Vorzugsweise sind die Kontaktanordnung und das Trägersubstrat über einen Fügebereich verbunden, der eine parallel zur Wickelachse orientierte Fügebereichslängserstreckung besitzt, die maximal der gegenseitigen Überlappung zwischen der Kontaktanordnung und dem Tubus in orthogonaler Projektion zur Wickelachse entspricht. Hierbei ist es besonders vorteilhaft, wenn die Längserstreckung der Kontaktanordnung gleich oder größer als die Tubuslänge ist.

Auch sind Ausführungsbeispiele denkbar, bei denen die Dimensionen der Längserstreckung der Kontaktanordnung, der Tubuslänge sowie der Fügebereichslängserstreckung voneinander abweichen, jedoch gilt es auch in diesen Fällen in vorteilhafter Weise darauf zu achten, dass die Kontaktanordnung relativ zum Trägersubstrat derart fest gefügt ist, so dass die Längserstreckung der Kontaktanordnung, die Tubuslänge sowie die Fügebereichslänge jeweils eine gemeinsame, orthogonal zur Wickelachse orientierte Mittelachse besitzen.

Die Kontaktanordnung weist einen plättchenförmigen Träger auf, an dem die wenigstens eine Cuff-Elektroden seitige elektrische Leitung mit einer auf dem Träger angebrachten Elektrode kontaktiert ist, vorzugsweise mittels eines Mikroflex-Kontaktes. Die Träger-seitige Elektrode ist wiederum mit einer weiteren separaten am Träger aufgebrachten Elektrodenfläche verbunden, an der die zur separaten Versorgungseinheit führende, elektrische Zu- und/oder Ableitung im Wege einer Schweiß-, Löt- oder Bondverbindung elektrisch verbunden ist. Selbstverständlich befindet sich an dem plättchenförmigen Träger eine Vielzahl derartiger Elektroden/Elektrodenflächen-Paare, über die eine entsprechende Anzahl von Cuff-Elektroden-seitigen elektrischen Leitungen mit entsprechenden zu einem Kabelstrang zusammengefasste elektrischen Zu- und/oder Ableitungen verbunden sind.

Die elektrische Zu- und/oder Ableitung bzw. die Vielzahl zu einem Kabelstrang zusammengefassten elektrischen Zu- und Ableitungen samt des plättchenförmigen Trägers ist von einem elastischen Material umschlossen, das im Fügebereich fluiddicht an das Trägersubstrat gefügt ist. Das elastische Material, das die wenigstens eine elektrische Zu- und/oder Ableitung fluiddicht, schlauch- oder matrixförmig umgibt, bildet die Form eines Stranges mit einer Stranglängserstreckung, deren Länge nach Maßgabe der intrakorporalen separaten Verortung von Cuff-Elektrode und Versorgungseinheit bestimmt ist.

Der plättchenförmige Träger weist eine gegenüber seiner Trägerbreite sehr viel größer bemessene Trägerlänge auf, deren Längserstreckung parallel zur Wickelachse orientiert ist. Die verhältnismäßig schmale Trägerform ist vorzugsweise derart dimensioniert, so dass sie ohne oder ohne wesentliche Formänderung innerhalb des Stranges, längs dem die wenigstens eine elektrische Zu- und/oder Ableitung integriert ist, eingebettet ist. Eine konkrete Ausführungsform ist nachstehend näher erläutert.

In einer bevorzugten Ausführungsform ist zu Zwecken einer zusätzlichen Kraftverteilung der über die Cuff-Elektrode auf das Nervenfaserbündel einwirkenden Kräfte wenigstens ein zusätzliches Befestigungsmittel, in Art einer Wickelmanschette oder einer Helikalstruktur, benachbart zur Cuff-Elektrode längs des die wenigstens eine elektrische Zu- und/oder Ableitung enthaltenen Stranges oder Schlauches angebracht. Das zusätzliche Befestigungsmittel umfasst gleichsam der Cuff-Elektrode einen geradzylinderförmigen Hohlraum, der koaxial zur Wickelachse ausgerichtet ist.

In einer weiteren bevorzugten Ausführungsform sieht der Strang bzw. Schlauch in Stranglängserstreckung eine an die Kontaktanordnung anschließende Strangverlängerung vor, an der mittel- oder unmittelbar ein zweites Befestigungsmittel nach Art des vorstehend ersten Befestigungsmittels ausgebildet und angebracht ist. Auf diese Weise können die längs der Zu- und/oder Ableitung wirkenden Zug- bzw. Schubkräfte symmetrisch zur Kraft-Elektrodenanordnung längs des Nervenstranges verteilt um- bzw. weitergeleitet werden.

Sämtliche auf dem Trägersubstrat aufgebrachte elektrische Leiterstrukturen, umfassend jeweils die wenigstens eine Elektrodenoberfläche sowie die wenigstens mit der Elektrodenoberfläche elektrisch verbundene elektrische Leitung, sind jeweils einstückig, vorzugsweise im Rahmen eines Metallabscheidungsverfahrens hergestellt, so dass keinerlei Fügestellen und damit verbundene unstete elektrische Impedanzsprünge längs der elektrischen Leiterstruktur vorhanden sind.

### Kurze Beschreibung der Erfindung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigt:

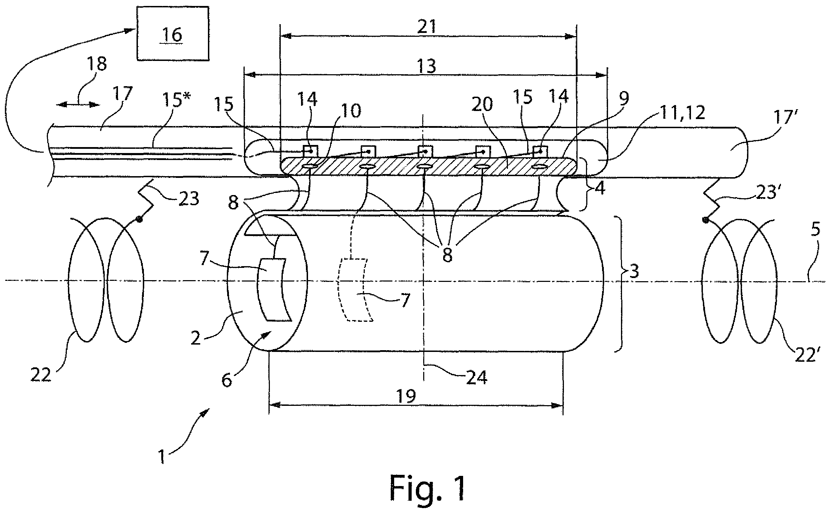

- Fig. 1: Ausführungsbeispiel eines lösungsgemäß ausgebildeten medizinischen Implantats.

### Wege zur Ausführung der Erfindung, gewerbliche Verwendbarkeit

Figur 1 zeigt eine in Art einer Wickelmanschetten-Elektrodenanordnung ausgebildete Cuff-Elektrode 1, die ein folienartig ausgebildetes Trägersubstrat 2 vorsieht, das vorzugsweise aus einer Polyimid-Folie besteht. Das Trägersubstrat 2 weist zwei einstückig zusammenhängende Bereiche 3, 4 auf, von denen der erste Bereich 3 des Trägersubstrates 2 um eine Wickelachse 5 aufgewickelt ist. Der zu einem Tubus aufgewickelte erste Bereich 3 des Trägersubstrats 2 behält seine aufgewickelte Form aufgrund materialinhärenter Spannkräfte, die durch eine geeignete Behandlung des folienartigen Trägersubstrates 2, beispielsweise im Wege einer Wärmebehandlung, eingeprägt sind, und umfasst einen geradzylinderförmigen Hohlraum 6, der axial beidseitig offen ist. Auf der dem geradzylinderförmigen Hohlraum 6 zugewandten Oberfläche des tubusartig aufgewickelten ersten Bereiches 3 des Trägersubstrates 2 ist wenigstens eine Elektrodenfläche 7, vorzugsweise eine Vielzahl derartiger Elektrodenflächen, aufgebracht, die jeweils die Außenwand eines nicht weiter dargestellten Nervenfaserbündels unmittelbar körperlich kontaktiert. Jede der Elektrodenflächen 7 ist einstückig mit einer elektrischen Leitung 8, die allesamt elektrisch isoliert innerhalb des Trägersubstrats 2 verlaufen, kontaktiert.

Unmittelbar der sich einstückig an den ersten Bereich 3 des Trägersubstrates 2 anschließende zweite Bereich 4 des Trägersubstrates weist eine dem ersten Bereich 3 abgewandte Seitenkante 9 auf, längs der die elektrischen Leitungen 8 nebeneinanderliegend angeordnet enden und jeweils über einen Mikroflexkontakt 10 mit einer auf der Kontaktanordnung 11 angebrachten Elektrode kontaktiert sind. Die Kontaktanordnung 11 ist in Form eines plättchenförmigen Trägers 12, vorzugsweise bestehend aus einer Keramikplatte ausgebildet, und verfügt über eine Längserstreckung 13. Die Länge des Trägers 12 ist sehr viel größer als dessen Breite ausgebildet.

Die an dem plättchenförmigen Träger 12 angebrachten Elektroden verbinden die elektrischen Leitungen 8 jeweils mit einer auf der Oberseite des plättchenförmigen Trägers 12 aufgebrachten elektrischen Kontaktfläche 14, auf der im Wege einer Löt-Bond- oder Schweißverbindung eine elektrische Zu- und/oder Ableitung 15 elektrisch kontaktiert ist, die seitlich zur Kontaktanordnung 11, in der Bilddarstellung gemäß Figur 1 nach links abführt. Sämtliche mit den jeweiligen Kontaktflächen 14 verbundenen Zu- und Ableitungen 15 werden zu einem Kabelstrang 15* zusammengefasst, der zu einer separat zur Cuff-Elektrode 1 ausgebildeten Versorgungseinheit 16 führt, die beispielsweise Steuersignale sowie auch elektrische Energie zum Betrieb der Cuff-Elektrode 1 zur Verfügung stellt. Die jeweils zu einem Kabelstrang 15* zusammengefassten drahtförmigen Zu- und Ableitungen 15 sind für eine separate Handhabung der Versorgungseinheit 16, bspw. zum Zwecke eines Austausches, über eine fluiddichte Steckverbindung, nicht dargestellt, mit der Versorgungseinheit 16 verbunden. Zur elektrischen Isolation sowie auch zum Zwecke des Schutzes gegenüber dem feuchten intrakorporalen Milieu ist der sämtliche elektrische Zu- und/oder Ableitungen 15 umfassende Kabelstrang 15* von einem Silikonschlauch 17 umgeben bzw. in einem Silikonstrang 17 eingebettet.

Um zu gewährleisten, dass längs des Silikonstrangs 17 wirkende Zug- oder Schubkräfte 18 möglichst gleich verteilt längs der gesamten Länge 19 des zu einem Tubus aufgewickelten ersten Bereiches 3 des Trägersubstrates 2 wirken, ist der Fügebereich 20 zwischen der Kontaktanordnung 11 und dem zweiten Bereich 4 des Trägersubstrates 2, in dem das Trägersubstrat 2 im Wege einer festen Fügeverbindung an die Oberfläche des plättchenförmigen Trägers 12 der Kontaktanordnung 11 gefügt ist, in orthogonaler Projektion zur Wickelachse 5 wenigstens teilweise, vorzugsweise vollständig, wie in Figur 1 dargestellt, überlappend zur axialen Erstreckung 19 des zu einem Tubus gewickelten Bereiches 3 des Trägersubstrates 2 angeordnet. Unabhängig von der tatsächlichen Bemessung der jeweiligen Längen der Kontaktanordnung 11, des Fügebereiches 20 sowie des zu einem Tubus gewickelten ersten Bereiches 3 des Trägersubstrates 2, besitzen die Längserstreckung (13) der Kontaktanordnung (11), die Tubuslänge (19) sowie die Fügebereichslänge (21) vorzugsweise jeweils eine gemeinsame orthogonal zur Wickelachse (5) orientierte Mittelachse (24).

Im Falle der Figur 1 weist der Fügebereich 20 eine Längserstreckung 21 auf, die in etwa der Länge 19 des tubusförmig gewickelten Abschnitts 3 des Trägersubstrats 2 entspricht. In diesem Fall erfolgt ein Kraftübertrag seitens der Kontaktanordnung 11 über den Fügebereich 20 auf das Trägersubstrat 2 gleichmäßig über die gesamte Länge 19.

Optional bietet es sich an einseitig zur Cuff-Elektrode 1 oder beidseitig zur Cuff-Elektrode 1 längs der Wickelachse 5 jeweils ein Befestigungsmittel 22, 22' vorzusehen, das sich gleichsam wie die Cuff-Elektrode 1 kraftbeaufschlagt um den Außenumfang eines Nervenstranges zu schmiegen vermag. Das Befestigungsmittel 22, 22' kann in Form einer Wickelmanschette oder einer an sich bekannten Helikalstruktur, vorzugsweise aus einem Silikonmaterial gefertigt sein. Das in der Bilddarstellung linke Befestigungsmittel 22 ist unmittelbar am Silikonstrang 17 über eine Verbindung 23 angebracht. Die Verbindung 23 ist vorzugsweise als einstückige Stoffschlussverbindung ausgebildet, d.h. das Befestigungsmittel 22, die Verbindung 23 sowie der Silikonstrang 17 sind aus jeweils gleichem Material im Rahmen eines einheitlichen Herstellungsprozess gefertigt. Die Verbindung 23 kann in Form eines nahtlosen Überganges zwischen Silikonstrang 17 und Befestigungsmittel 22 bis hin zu einer räumlichen Fügegeometrie ausgebildet sein, bspw. in Form eines geradlinigen, zick-zack, wellig, spiral oder helikal geformten Verbindungsarmes. Das ebenfalls optional vorgesehen Befestigungsmittel 22' zur rechten Seite der Cuff-Elektrode 1 ist an einer Strangverlängerung 17' angebracht. Auch in diesem Fall kann die Verbindung 23' in der gleichen Weise, wie vorstehend beschrieben, ausgebildet sein.

### Bezugszeichenliste

- 1: Cuff-Elektrode
- 2: Trägersubstrat
- 3: Erster Bereich des Trägersubstrats
- 4: Zweiter Bereich des Trägersubstrats
- 5: Wickelachse
- 6: Geradzylinderförmiger Hohlraum
- 7: Elektrodenoberfläche
- 8: Elektrischer Leiter
- 9: Verbindungsendkante des Trägersubstrates
- 10: Mikroflex-Kontakt
- 11: Kontaktanordnung
- 12: plättchenförmiger Träger
- 13: Längserstreckung der Kontaktanordnung
- 14: Elektrodenfläche
- 15: Elektrische Zu- und/oder Ableitung
- 15*: Kabelstrang
- 16: Versorgungseinheit
- 17: Silikonstrang
- 17': Strangverlängerung
- 18: Schub-Zugkräfte
- 19: Länge des zu einem Tubus gewickelten zweiten Bereiches 3 des Trägersubstrates
- 20: Fügebereich
- 21: Länge des Fügebereiches
- 22,22': Befestigungsmittel
- 23, 23': Verbindung
- 24: Mittelachse

## Patentansprüche

1. Medizinisches Implantat in Art einer Wickelmanschetten-Elektrodenanordnung, kurz Cuff-Elektrode (1), die ein flexibles, biokompatibles, folienartiges Trägersubstrat (2) aufweist, das in einem ersten Bereich (3) durch Wickeln um eine Wickelachse (5) die Gestalt eines Tubus mit einer Tubuslänge (19) annimmt, der einen geraden, zylinderförmigen Hohlraum (6) umfasst, der von einer Oberfläche des Trägersubstrats radial zur Wickelachse (5) begrenzt ist, an der wenigstens eine Elektrodenoberfläche (7) angebracht ist, die über wenigstens eine innerhalb des Trägersubstrates (2) integrierten elektrischen Leitung (8) mit einer nicht flexiblen Kontaktanordnung (11) verbunden ist, an der die elektrische Leitung (8) mit einer elektrischen Zu- und/oder Ableitung (15) verbunden ist, die zu einer zum Implantat separat ausgebildeten, implantierbaren elektrischen Versorgungseinheit (16) führt, wobei die die Kontaktanordnung (11) eine räumliche Längserstreckung (13) besitzt, die parallel zur Wickelachse (5) orientiert ist, und wobei die Kontaktanordnung (11) mit dem Trägersubstrat (2) längs eines formstabilen Fügebereiches (20) fest gefügt ist, der eine parallel zur Wickelachse (5) orientierte Fügebereichslänge (21) besitzt, und wobei die Kontaktanordnung (11) in orthogonaler Projektion zur Wickelachse (5) mit dem zu einem Tubus gewickelten ersten Bereich (3) des Trägersubstrates (2) überlappt, **dadurch gekennzeichnet dass** die Kontaktanordnung (11) einen plättchenförmigen Träger (12) aufweist, an dem die wenigstens eine elektrische Leitung (8) mittels eines elektrischen Kontaktes (15) kontaktiert ist, der elektrisch mit jeweils einer auf dem plättchenförmigen Träger (12) aufgebrachten Elektrode (14) verbunden ist, mit der die elektrische Zu- und/oder Ableitung (15) elektrisch verbunden ist, und dass die elektrische Zu- und/oder Ableitung (15) samt des plättchenförmigen Trägers (12) von einem elastischen Material umschlossen ist, das im Fügebereich (20) fluiddicht an das Trägersubstrat (2) gefügt ist.

2. Medizinisches Implantat nach Anspruch 1,
**dadurch gekennzeichnet, dass** die Kontaktanordnung (11) relativ zum Trägersubstrat (2) derart angeordnet ist, dass sich die Längserstreckung (13) der Kontaktanordnung (11) sowie die dem zu einem Tubus gewickelten ersten Bereich (3) des Trägersubstrates (2) zugeordnete Tubuslänge (19) in orthogonaler Projektion zur Wickelachse (5) maximal überlappen.

3. Medizinisches Implantat nach Anspruch 1 oder 2,
**dadurch gekennzeichnet, dass** der Fügebereich (20) zwischen der Kontaktanordnung (11) und dem Trägersubstrat (2) eine parallel zur Wickelachse (5) orientierte Fügebereichslänge (21) besitzt, die maximal einer gegenseitigen Überlappung der Längserstreckung (13) der Kontaktanordnung (11) und der Tubuslänge (19) in orthogonaler Projektion zur Wickelachse (5) entspricht.

4. Medizinisches Implantat nach einem der Ansprüche 1 bis 3,
**dadurch gekennzeichnet, dass** die Kontaktanordnung (11) relativ zum Trägersubstrat (2) derart fest gefügt ist, so dass die Längserstreckung (13) der Kontaktanordnung (11), die Tubuslänge (19) sowie die Fügebereichslänge (21) jeweils eine gemeinsame orthogonal zur Wickelachse (5) orientierte Mittelachse (24) besitzen.

5. Medizinisches Implantat nach einem der Ansprüche 1 bis 4,
**dadurch gekennzeichnet, dass** das Trägersubstrat (2) einstückig angrenzend an den ersten, zum Tubus gewickelten Bereich (3) einen zweiten flächigen Trägersubstratbereich (4) mit einer das Trägersubstrat (2) seitlich begrenzenden Seitenkante (9) besitzt, an der die wenigstens eine elektrische Leitung (8) innerhalb des Fügebereiches (20) mit der Kontaktanordnung (11) elektrisch verbunden ist.

6. Medizinisches Implantat nach einem der Ansprüche 1 bis 5,
**dadurch gekennzeichnet, dass** die wenigstens eine elektrische Leitung (8) mittels eines Mikroflex-Kontaktes kontaktiert ist.

7. Medizinisches Implantat nach einem der Ansprüche 1 bis 6,
**dadurch gekennzeichnet, dass** das die wenigstens eine elektrische Zu- und/oder Ableitung (15) umschließende elastische Material in Art eines Stranges (17) ausgebildet ist, mit einer Stranglängserstreckung, die zumindest in einem Bereich, der an die Kontaktanordnung (11) unmittelbar angrenzt, parallel zur Wickelachse (5) orientiert ist, und dass im Bereich des die wenigstens eine elektrische Zu- und/oder Ableitung (15) enthaltenen Stranges (17) mittel- oder unmittelbar ein, einen geradzylinderförmigen Hohlraum umfassendes erstes Befestigungsmittel (22), in Art einer Wickelmanschette oder einer Helikalstruktur, angebracht ist, dessen geradzylinderförmiger Hohlraum koaxial zur Wickelachse (5) orientiert ist.

8. Medizinisches Implantat nach Anspruch 7,
**dadurch gekennzeichnet, dass** der Strang (17) in Stranglängserstreckung gegenüberliegend zur Kontaktanordnung (11) eine Strangverlängerung (17') vorsieht, an der mittel- oder unmittelbar ein zweites Befestigungsmittel (22') nach Art des ersten Befestigungsmittels (22) angebracht ist.

9. Medizinisches Implantat nach einem der Ansprüche 1 bis 8,
**dadurch gekennzeichnet, dass** der erste zum Tubus gewickelte Bereich (3) des Trägersubstrates (2) wenigstens eine Wicklung um die Wickelachse aufweist, die zumindest einen Wicklungsbereich besitzt, in dem sich das Trägersubstrat (2) lose aneinander liegend radial zur Wickelachse (5) mit sich selbst überlappt.

## Claims

1. Medical implant in the form of a wrap-round cuff electrode arrangement, in short cuff electrode (1), which comprises a flexible, biocompatible film-like carrier substrate (2), which in a first area (3) through wrapping around a wrap axis (5), assumes the shape of a tube with a tube length (19), which contains a straight cylindrical hollow space (6) which is delimited by a surface of the carrier substrate radially to the wrap axis (5), on which at least one electrode surface (7) is applied, which is connected via at least one electrical lead (8) integrated within the carrier substrate (2), to a non-flexible contact arrangement (11), on which the electrical lead (8) is connected with an electrical inlet and/or outlet (15) which leads to an implantable electrical supply unit (16) designed separately from the implant,
wherein the contact arrangement (11) has a spatial longitudinal dimension (13), which is orientated in parallel to the wrap axis (5), and
wherein the contact arrangement (11) is firmly jointed to the carrier substrate (2) along a dimensionally stable joint area (20), which has a joint area length (21) orientated in parallel to the wrap axis (5), and wherein in orthogonal projection to the wrap axis (5), the contact arrangement (11) overlaps with the first area (3) of the carrier substate (2) wrapped to form a tube,
**characterised in that**
the contact arrangement (11) comprises a plate-shaped carrier (12) which is contacted by the at least one electrical lead (8) by means of an electrical contact (15), which is electrically connected to respectively one of the electrodes (14) applied to the plate-like carrier (12) with which the electrical inlet and/or outlet (15) is electrically connected, and **in that** the electrical inlet and/or outlet (15) together with the plate-like carrier (12) is surrounded by an elastic material which in the joint area (20) in jointed to the carrier substrate (2) in a fluid-tight manner.

2. Medical implant according to claim 1
**characterised in that** the contact arrangement (11) is arranged relative to the carrier substrate (2) in such a way that the longitudinal dimension (13) of the contact arrangement (11) as well as the tube length (19) of the first area (3) of the carrier substrate (2) wrapped form a tube, maximally overlap in orthogonal projection to the wrap axis (5).

3. Medical implant according to claim 1 or 2 **characterised in that** between the contact arrangement (11) and the carrier substrate (2), the joint area (20) has a joint area length (21) orientated in parallel to the wrap axis (5) that maximally corresponds with a mutual overlapping of the longitudinal dimension (13) of the contact arrangement (11) and the tube length (19) in orthogonal projection to the wrap axis (5).

4. Medical implant according to any one of claims 1 to 3 **characterised in that** the contact arrangement (11) is firmly jointed relative to the carrier substrate (2) in such a way that the longitudinal dimension (13) of the contact arrangement (11), the tube length (19) and the joint area length (21) each have a common central axis (24) orientated orthogonally to the wrap axis (5).

5. Medical implant according to any one of claims 1 to 4 **characterised in that**, adjoining, in one piece, the first area (3) wrapped to form a tube, the carrier substrate (2) comprises a second, flat carrier substance area (4) with a side edge (9), laterally delimiting the carrier substrate (2), to which the at least one electrical lead (8) is electrically connected to the contact arrangement (11) within the joint area (20) .

6. Medical implant according to any one of claims 1 to 5 **characterised in that** the at least one electrical lead (8) is contacted by mean of a microflex contact.

7. Medical implant according to any one of claims 1 to 6 **characterised in that** the elastic material encompassing a electrical inlet and/or outlet (15), is designed in the form of a strand (17) with a strand longitudinal dimension, which at least in an area that directly adjoins the contact arrangement (11), is orientated in parallel to the wrap axis (5), and **in that** in this area of the strand (17) containing the at least one electrical inlet and/or outlet (15), directly or indirectly, a first fastening means (22), in the form of a wrapping cuff or a helical structure and containing a straight cylindrical hollow space, is applied, the straight cylindrical hollow space of which is orientated coaxially to the wrap axis (5).

8. Medical implant according to claim 7
**characterised in that** the strand (17), in the strand longitudinal dimension opposite the contact arrangement (11), envisages a strand extension (17'), to which a second fastening means (22') of the type of the first fastening means (22) is directly or indirectly applied.

9. Medical implant according to any one of claims 1 to 8 **characterised in that** the first area (3) of the carrier substrate (2) wrapped to form a tube, comprises at least one wrapping around the wrap axis which has at least one wrapping area in which the carrier substrate (2) overlaps with itself in loose contact radially to the wrap axis (5).

## Revendications

1. Implant médical comprenant un arrangement d'électrodes de type à manchette, en bref, électrode manchette (1), qui comporte un substrat porteur (2) souple, biocompatible, de type film, qui adopte dans une première zone (3) par enroulement autour d'un axe d'enroulement (5), la forme d'un tube avec une longueur de tube (19), qui comprend un espace creux (6) droit, de forme cylindrique, qui est limité radialement par rapport à l'axe d'enroulement (5) par une surface du substrat porteur sur laquelle est placée au moins une surface d'électrode (7), qui est reliée par le biais d'au moins un conducteur électrique (8) intégré à l'intérieur du substrat porteur (2) à un système de contact (11) non souple, sur lequel le conducteur électrique (8) est relié à une ligne d'arrivée et/ou de départ électrique (15), qui conduit à une unité d'alimentation (16) électrique, implantable, constituée séparément à l'implant,
sachant que le système de contact (11) possède une extension longitudinale (13) spatiale, qui est orientée parallèlement à l'axe d'enroulement (5) et
sachant que le système de contact (11) est solidement joint au substrat porteur (2) le long d'une zone d'assemblage (20) de forme stable, qui possède une longueur de zone d'assemblage (21) orientée parallèlement à l'axe d'enroulement (5), et
sachant que le système de contact (11) se chevauche en projection orthogonale par rapport à l'axe d'enroulement (5) avec la première zone (3) du substrat porteur (2) enroulée à un tube,
**caractérisé en ce que**
le système de contact (11) comporte un support (12) en forme de plaquette sur lequel est mis en contact au moins un conducteur électrique (8) au moyen d'un contact électrique (15), qui est relié électriquement à respectivement une électrode (14) montée sur le support (12) en forme de plaquette à laquelle est reliée électriquement la ligne d'arrivée et/ou de départ électrique (15), et
**en ce que** la ligne d'arrivée et/ou de départ électrique (15) avec le support (12) en forme de plaquette est entourée d'un matériau élastique, qui est assemblé de façon hermétique aux fluides au substrat porteur (2) dans la zone d'assemblage (20).

2. Implant médical selon la revendication 1,
**caractérisé en ce que** le système de contact (11) est disposé par rapport au substrat porteur (2) de telle manière que l'extension longitudinale (13) du système de contact (11) et la longueur de tube (19) attribuée à la première zone (3) du substrat porteur (2), enroulée au tube, se chevauchent en projection orthogonale par rapport à l'axe d'enroulement (5).

3. Implant médical selon la revendication 1 ou 2, **caractérisé en ce que** la zone d'assemblage (20) entre le système de contact (11) et le substrat porteur (2) possède une longueur de zone d'assemblage (21) orientée parallèlement à l'axe d'enroulement (5), qui correspond au maximum à un chevauchement réciproque de l'extension longitudinale (13) du système de contact (11) et à la longueur de tube (19) en projection orthogonale par rapport à l'axe d'enroulement (5).

4. Implant médical selon l'une quelconque des revendications 1 à 3,
**caractérisé en ce que** le système de contact (11) par rapport au substrat porteur (2) est solidement joint de telle manière que l'extension longitudinale (13) du système de contact (11), la longueur de tube (19) ainsi que la longueur de la zone d'assemblage (21) possèdent respectivement un axe médian (24) commun orienté orthogonalement à l'axe d'enroulement (5).

5. Implant médical selon l'une quelconque des revendications 1 à 4,
**caractérisé en ce que** le substrat porteur (2) possède attenante en une pièce à la première zone (3) enroulée au tube, une deuxième zone de substrat porteur (4) de surface avec un bord latéral (9) délimitant latéralement le substrat porteur (2), sur lequel au moins un conducteur électrique (8) à l'intérieur de la zone d'assemblage (20) est relié électriquement au système de contact (11).

6. Implant médical selon l'une quelconque des revendications 1 à 5,
**caractérisé en ce qu'**au moins un conducteur électrique (8) est mis en contact au moyen d'un contact Microflex.

7. Implant médical selon l'une quelconque des revendications 1 à 6,
**caractérisé en ce que** le matériau élastique entourant au moins une ligne d'arrivée et/ou de départ électrique (15) est constitué à la manière d'un cordon (17) avec une extension longitudinale de cordon, qui est orientée, au moins dans une zone, qui est directement adjacente au système de contact (11), parallèlement à l'axe d'enroulement (5) et **en ce que** dans la zone du cordon (17) contenant au moins une ligne d'arrivée et/ou de départ (15) électrique, est placé indirectement ou directement un premier moyen de fixation (22) comprenant un espace creux de forme cylindrique droite, de type d'une manchette d'enroulement ou d'une structure hélicoïdale, dont l'espace creux de forme cylindrique droite est orienté de façon coaxiale par rapport à l'axe d'enroulement (5).

8. Implant médical selon la revendication 7,
**caractérisé en ce que** le cordon (17) prévoit dans l'extension longitudinale de cordon, une rallonge de cordon (17') opposée au système de contact (11), sur laquelle est placé indirectement ou directement un deuxième moyen de fixation (22') à la manière du premier moyen de fixation (22).

9. Implant médical selon l'une quelconque des revendications 1 à 8,
**caractérisé en ce que** la première zone (3) du substrat porteur (2), enroulée au tube, comporte au moins un enroulement autour de l'axe d'enroulement, qui possède au moins une zone d'enroulement dans laquelle le substrat porteur (2) se chevauche lui-même appliqué non serré, radialement par rapport à l'axe d'enroulement (5) .
